(19) **Europäisches Patentamt**
**European Patent Office**
**Office européen des brevets**

(11) **EP 4 017 960 B1**

(12) # EUROPEAN PATENT SPECIFICATION

(45) Date of publication and mention
of the grant of the patent:
**17.06.2026 Bulletin 2026/25**

(21) Application number: **20756866.8**

(22) Date of filing: **18.08.2020**

(51) International Patent Classification (IPC):
*C12N 5/00* (2006.01)     *G01N 21/552* (2014.01)

(52) Cooperative Patent Classification (CPC):
**G01N 21/658; C12N 5/0075;** G01N 21/554

(86) International application number:
**PCT/EP2020/073104**

(87) International publication number:
**WO 2021/032743 (25.02.2021 Gazette 2021/08)**

(54) **MICROCARRIERS FOR CELL CULTURE**

MIKROTRÄGER FÜR ZELLKULTUR

MICROPORTEURS POUR CULTURE CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.08.2019 LU 101355**

(43) Date of publication of application:
**29.06.2022 Bulletin 2022/26**

(73) Proprietor: **Luxembourg Institute of Science and
Technology (LIST)
4362 Esch-sur-Alzette (LU)**

(72) Inventors:
• **KRISHNAMOORTHY, Sivashankar
4362 Esch-sur-Alzette (LU)**
• **STOFFELS, Charlotte
4362 Esch-sur-Alzette (LU)**

(74) Representative: **Aronova
Aronova S.A.
BP 327
12, avenue du Rock'n'Roll
4004 Esch-sur-Alzette (LU)**

(56) References cited:
**WO-A1-2014/198989     KR-A- 20160 020 160
US-A1- 2019 249 136**

• **BERNAT MIR-SIMON ET AL: "SERS efficiencies
of micrometric polystyrene beads coated with
gold and silver nanoparticles: the effect of
nanoparticle size", JOURNAL OF OPTICS,
INSTITUTE OF PHYSICS PUBLISHING, BRISTOL
GB, vol. 17, no. 11, 23 October 2015 (2015-10-23),
pages 114012, XP020291791, ISSN: 2040-8986,
[retrieved on 20151023], DOI: 10.1088/2040-8978/
17/11/114012**
• **"Stem Cell Manufacturing", 1 January 2016,
ELSEVIER, ISBN: 978-0-444-63265-4, article S.M.
BADENES ET AL: "Microcarrier Culture Systems
for Stem Cell Manufacturing", pages: 77 - 104,
XP055494887, DOI: 10.1016/
B978-0-444-63265-4.00004-2**

## Description

### Field of the Invention

The invention generally relates to microcarriers for cell culture, to a method of production of such microcarriers and to use thereof.

### Background of the Invention

Microcarriers (MCs) with dimensions in the order of few tens of microns have been used as three-dimensional cell-culture growth surfaces, enabling cell-expansion with advantages of better nutrient availability, enhanced surface areas, and automated and high-throughput harvesting within controlled environments within bioreactors.

MCs made of organic and inorganic materials and presenting different surface chemistries are commercially available, even as research in the area have focused on newer MCs that could be engineered to provide chemical, mechanical cues to influence cell proliferation, maintaining stemness of stem cells, promoting stem-cell differentiation into desired cell- type and to enable easy release of cells.

### Summary of the Invention

Beyond the above goals, an additional significant advance to the use of MCs for cell expansion would be to integrate sensing functions that can potentially help monitor cell health during cell culture, and/or to report biomarkers of interest.

According to a first aspect of the invention, a cell culture microcarrier bead is proposed. The cell culture microcarrier bead (10), comprises a bead body (12) having its surface flecked with plasmonic nanoparticles (14), the bead body having a diameter in the range from 50 $\mu$m to 1 mm, the bead body comprising a swollen or unswollen hydrogel.

As used herein, the term "nanoparticle" designates a particle having a surrounding interfacial layer and with a characteristic dimension in the sub-micrometer range, e.g. with diameter in the range from 1 to 500 nm Preferably, the plasmonic metal nanoparticles have a diameter in the range from 2 to 200 nm. In the present document and unless contradicted by context, the term "diameter" means the smallest distance that can be formed between two opposite parallel planes tangent to the convex hull of the object under consideration. The measurement of diameters may be made, e.g., by scanning electron microscopy (SEM), transmission electron microscopy or scanning probe microscopy (SPM).

Preferably, the plasmonic nanoparticles comprise or consist of metal nanoparticles. Such plasmonic metal nanoparticles could comprise or consist of, e.g., gold, silver or aluminium nanoparticles but other plasmonic metals or alloys are not excluded. The plasmonic nanoparticles could be functionalized, e.g. such that they would bind specifically to receptors on a cell.

The bead body comprises a hydrogel matrix in a swollen (hydrated) or unswollen (dehydrated) state. Such a hydrogel matrix could comprise or consist of, e.g., polysaccharides (such as, e.g., agarose, dextran, etc.), elastin-like polypeptides (ELP), or the like. The bead body could consist of such a hydrogel matrix or, alternatively, have a (solid) core surrounded by the hydrogel matrix.

The bead body preferably carries positively charged groups distributed within its volume. In this case, attachment of the plasmonic nanoparticles to the bead bodies may be effected by electrostatic assembly.

Preferably, the microcarrier bead carries the plasmonic nanoparticles only on the bead body surface, i.e. not in the bead body interior, at least, not deep in the bead body interior. "Deep" means, in this context, 0.3·R or more from the surface, with R designating the bead body radius.

The plasmonic nanoparticles could be stabilized with a capping agent, e.g. citrate.

The plasmonic nanoparticles could be of any shape, e.g. spheres, stars, rods, cubes, etc. In case of spherical nanoparticles, their diameter is preferably less than or equal to 100 nm. Nanoparticles of other shapes, e.g., nanostars, nanorods, etc. should be larger in size. If the nanoparticles are nanorods, the cross-sectional diameter should preferably lie in the range from 2 to 200 nm while the length of the nanorods could be greater.

The body of the microcarrier bead has a diameter in the range from 50 $\mu$m to 1 mm. The ratio of the diameter of the bead body to the diameter of the plasmonic metal nanoparticles preferably lies in the range from 250 to 50000, more preferably in the range from 250 to 25000. In case of a bead body with a hydrogel matrix, the indicated diameter range may refer to the diameter in dehydrated state or to the diameter in hydrated state (e.g. when in use in the cell culture medium) or any intermediary state.

In a second aspect, the invention relates to a cell culture reactor, containing a cell culture medium and microcarrier beads as described herein.

A further aspect of the invention concerns a method for observing (in vitro) living cells on a microcarrier bead as described herein. The method comprises:

○ illuminating the microcarrier bead with probe light so as to excite surface plasmons in the plasmonic metal nanoparticles,

○ and detecting light emitted from the plasmonic metal nanoparticles in response to the illumination.

The detection is preferably based on one or more of the following techniques: (localized) surface plasmon resonance (SPR), surface-enhanced Raman spectroscopy (SERS), fluorescence and second-harmonic generation. According to the SPR technique, one measures changes in the absorption wavelength or any other property of the detected light that is indicative of local refractive index variations induced by cell reactions, e.g. interaction of the cell with another bioentity (e.g. an antigen), cell division, etc.

Yet a further aspect of the invention relates to a method for packing nanoparticles on a carrier body which is a cell culture microcarrier bead. The method comprises:

○ attaching nanoparticles on a surface of the carrier body while the carrier body is in a swollen state,

○ reducing the volume of the carrier body and thereby causing the nanoparticles to move closer together.

The attachment process itself may be effected using electrostatic attraction between the carrier body and the nanoparticles. It will be appreciated that such an electrostatic assembly may be considered a random-sequential adsorption (RSA) process that has a theoretical maximum coverage. Coverages attained in experiments are typically significantly lower than the theoretical maximum coverage, in particular because of limited time. It will thus be appreciated that the present method allows increasing the density of the nanoparticles by attaching them when the carrier body is swollen and then causing the carrier body to shrink. The smaller average distance between the nanoparticles in the shrunk state may be beneficial in practical applications, e.g. plasmonic sensing. Alternatively, the method could be used to shorten the attachment process for a given target density of the nanoparticles.

As indicated above, the nanoparticles are plasmonic nanoparticles, e.g. plasmonic metal nanoparticles.

The carrier body comprises a hydrogel matrix and the volume reduction is effected by (at least partial) dehydration of the hydrogel.

It will be appreciated that the presence of plasmonic nanoparticles on the microcarrier beads could be used e.g. for harvesting the cells. For instance, release of cells from the microcarrier bead could be stimulated by exciting localized surface plasmon resonance in the plasmonic nanoparticles. Specifically, the release of cells could be stimulated by plasmonic heating of the nanoparticles.

## Brief Description of the Drawings

The accompanying drawings illustrate several aspects of the present invention and, together with the detailed description, serve to explain the principles thereof. In the drawings:

Fig. 1: is a schematic of a cell culture microcarrier having its surface coated with plasmonic nanoparticles;
Fig. 2: is an illustration of a process for attaching plasmonic nanoparticles on a microcarrier for cell culture.
Fig. 3: is chart of the size distributions of plasmonic nanoparticles (left-hand side) and microcarriers (right-hand side) used in examples of implementation of the invention;
Fig. 4: shows SEM images of hydrogel microcarriers covered with different concentrations of gold nanoparticles and then dried;
Fig. 5: shows SEM images of microtomed slices of AuNP-coated microcarriers, focused on the interface between fused microcarriers, with magnification increasing from (i) to (iv);
Fig. 6: shows optical microscopy images of naked MCs in dry state (a), of naked MCs in water (c), of AuNP-coated MCs in water, and the corresponding observed size distributions (d);
Fig. 7: shows a photography of suspensions of Au nanoparticle coated microcarriers with increasing coverage (a)), a graph of the corresponding absorbance spectra (b)) and a graph of the peak intensities and positions (c));
Fig. 8: is a chart of the evolution of glucose and lactate concentrations in cell cultures on naked microcarriers and on plasmonic-nanoparticle-coated microcarriers.

## Description of Preferred Embodiments

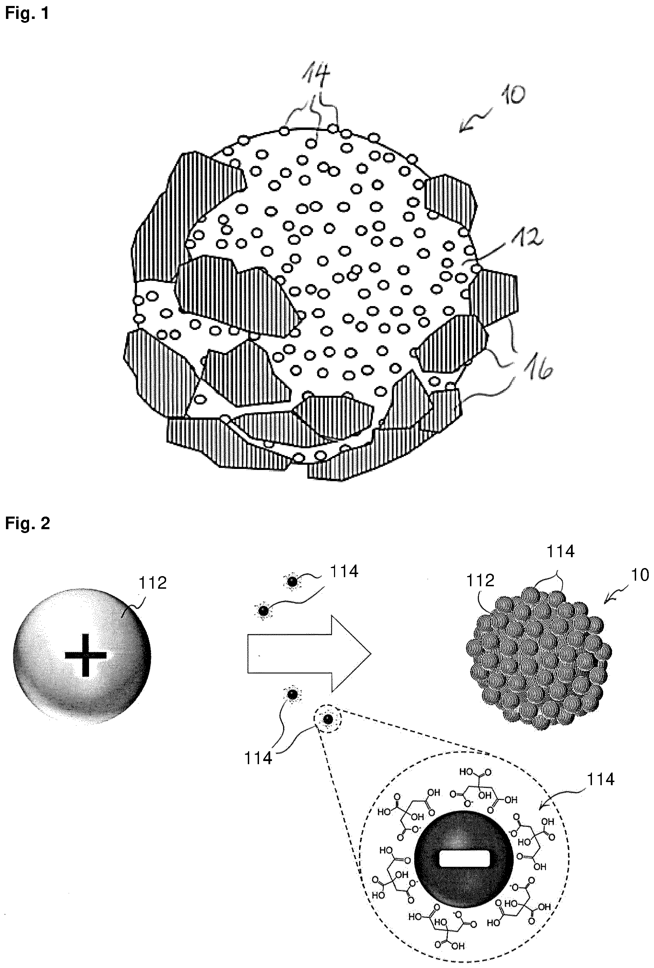

Fig. 1 schematically illustrates a cell culture microcarrier bead 10. The microcarrier bead 10 comprises a bead body 12 (e.g. a conventional, commercially available MC) having its surface flecked with plasmonic nanoparticles 14. Also illustrated are cells 16 that use the microcarrier bead 10 as a growth surface.

Regarding the integration of sensing functions, such as, e.g., cell health monitoring and/or biomarker reporting, into microcarrier beads, it is a key requirement that the modification of MCs does not have an uncontrolled adverse impact on cell adhesion and proliferation and that the sensing function(s) are compatible with the environment, in which the MCs are to be used. Therefore, when microcarrier beads with attached plasmonic nanoparticles are to be used, comparative tests should be carried out in order to assess whether (and, if necessary, to what extent) the presence of the plasmonic nanoparticles changes the behaviour of the cultured cells.

In the examples discussed below, it is shown that the integration of high-density assemblies of gold nanoparticle on the surface of dextran-based MCs (commercially available Cytodex-1™ microcarriers based on a cross-linked dextran matrix that is substituted with positively charged N,N-diethylaminoethyl, DEAE, groups) retain the cell-expansion and cell-migration capabilities on par with those of the uncoated (unmodified) MCs. It is further demonstrated that the gold-nanoparticle-flecked MCs are suitable for plasmonic sensing. The results show that gold nanoparticles are an excellent choice to integrate onto microcarriers, given the biocompatibility of gold particles, ease of sensing using their unique optical, spectroscopic properties, while maintaining stability in complex environment.

As illustrated in Fig. 2, the attachment of gold nanoparticles 114 on MCs 112 may be effected by electrostatic self-assembly, taking advantage of the presence of positive charges within the polymer matrix of the MCs. Fig. 2 more specifically illustrates electrostatic self-assembly of citrate-stabilized gold nanoparticles 114 (see enhanced detail) on a positively charged hydrogel MC 112. Electrostatic self-assembly processes are known as such but, to the best knowledge of the inventors, have not been previously tried on MCs. Irreversible monolayer attachment of charged colloids to oppositely charged surfaces may be considered as a random-sequential adsorption (RSA) process that results in a theoretical maximum surface coverage of 54.7% (jamming limit), although the practically attained surface coverages are typically significantly lower.

MCs flecked with plasmonic nanoparticles are useful, e.g., for sensing based on surface-enhanced Raman spectroscopy (SERS), taking advantage of the enhanced electromagnetic fields arising in close proximity of nanostructured surface. The magnitude of the enhancement depends on the geometric attributes of the features and their assemblies, and is non-linear with decreasing separation between neighboring nanoparticles. High electromagnetic fields at junctions between nanoparticles are termed "gap hot-spots". The likelyhood of occurrence of such hot spots increases with nanoparticle density. It may thus be particularly advantageous to assemble the plasmonic nanoparticle on hydrogel-based microcarriers in conditions (e.g., temperature, pH, etc.) wherein the latter are heavily swollen. By making the MCs shrink (by changing the environmental conditions), the mean distance between the nanoparticles attached to the surface is reduced, which favours the occurrence of the above-mentioned hot spots.

## Examples

DEAE-dextran MCs (Cytodex-1™, purchased from Sigma-Aldrich) were functionalized with citrate-stabilized gold nanoparticles by electrostatic interaction, as represented in Fig. 2. MilliQ water (18.2 M$\Omega$cm, Millipore) was used for the preparation of the solutions and for all rinses. Citrate-stabilized gold nanoparticles (AuNPs) were prepared according to the Turkevich method, by reduction of chloroauric acid (HAuCl$_4$, Sigma-Aldrich) with sodium citrate (Na$_3$C$_6$H$_5$O$_7$, Sigma-Aldrich). The obtained AuNPs were substantially spherical with an average size of 12 nm.

The prepared colloidal suspension was stored at 4°C and taken out from the refrigerator 12 hours before being used. Eppendorf Safe-Lock Tubes 2 ml, Conical Bottom Centrifuge Tubes 50 ml, Silicon Wafers (Siegert Wafer) and Glass Wafers (Menzel™ Microscope Coverslips) were used as containers and supports for characterization, respectively.

The size of the AuNPs was measured by Focused Ion Beam Scanning Electron Microscopy (FIB-SEM). FIB-SEM images of the AuNPs were obtained using Helios NanoLab™ 650 microscope operating at an accelerating voltage of 20 kV and a distance of 4 mm. The secondary electron detector was used. The AuNPs were adsorbed on positively charged silicon surface functionalized with 3-aminopropyl trimethoxy silane, then the size of the AuNPs was measured.

The size of the MCs was measured by optical microscopy. Images of the MCs were obtained using Olympus Microscope BX51. Two objectives (Olympus; x10, x50) were used to focus. Images were acquired using OLYMPUS Stream Image Analysis Software.

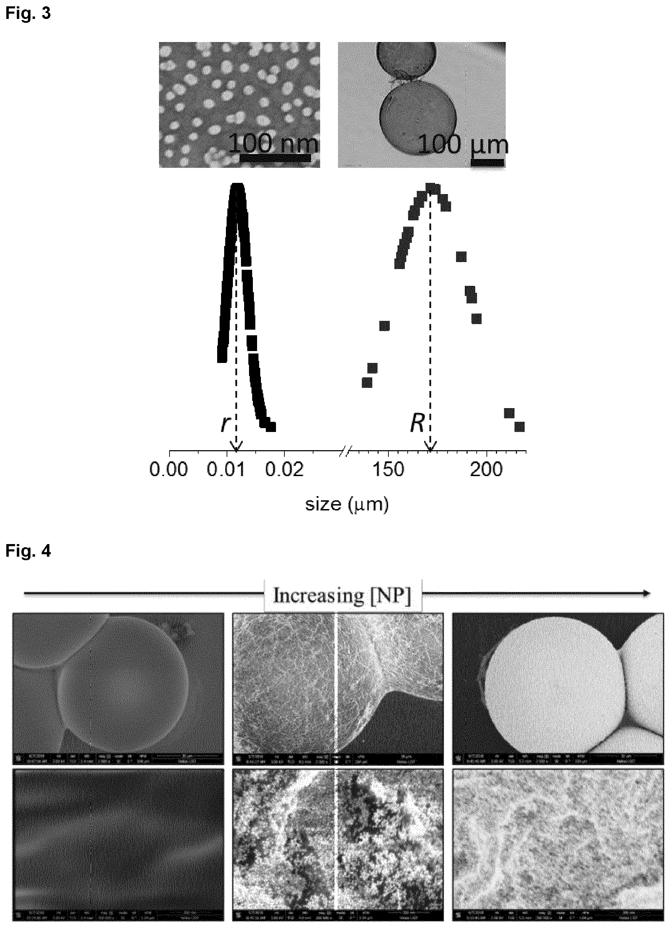

Fig. 3 shows the size distributions of the AuNPs (left) measured by FIB-SEM and of the AuNP-flecked MCs (right) in water.

Dynamic Light Scattering (DLS) was used to determine hydrodynamic size and zeta potential of the gold nanoparticles. The machine used was the MALVERN ZETASIZER NANO ZS. Disposable Folded Capillary Zeta Cell (MALVERN) was used for the measurement of both parameters. The pH of the AuNP solution was equal to 5.8. The sample was prepared as follows: 100% and 33% diluted solution of gold nanoparticles were injected inside two different disposable cells (for comparison). The zeta potential of the gold nanoparticles was determined by DLS to be equal to -34 ± 10 mV at pH 6. The zeta potential is negative due to the capping agent (citrate) which is negatively charged at that pH value. The microcarriers being too large, they could not be characterized by DLS. However, it is known that DEAE-dextran is positively charged at almost all pH due to its DEAE-groups. Consequently, the gold nanoparticles were expected to cover the surface of the microcarriers. Cytodex-1 is a cross-linked hydrogel having positive groups throughout the entire matrix. Thus, the question arises whether gold nanoparticles penetrate inside the microcarriers and functionalize the internal surfaces (pores). However, as detailed below, the characterization of external and internal structure reveals the distribution of gold nanoparticles to the outer surface only. DEAE-dextran microcarriers swell when added into any medium. Consequently, the surface area available for interaction with gold nanoparticles changes according to the medium used.

The distribution and number of gold nanoparticles per microcarrier was investigated by SEM. Fig. 4 shows SEM images of MCs covered with different concentrations of gold nanoparticles and then dried. The shrinking of the MCs during the drying process results in a wrinkled surface and a denser packing of the AuNPs. The AuNP coverage increases from left to right (shown at two magnifications). The transition from low to high concentration is easily observable. At saturated coverage, the microcarriers were found to be fully covered with nanoparticles, while at lower particle coverages, the nanoparticles show homogeneous, yet patchy coverage.

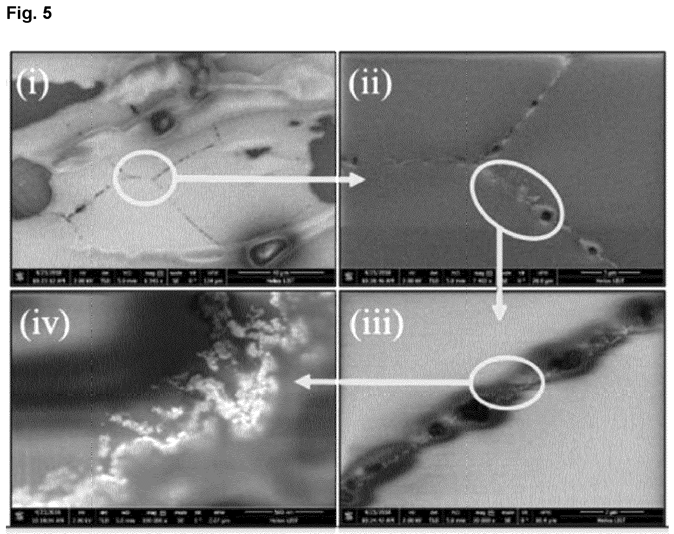

To investigate the questions whether gold nanoparticles penetrate into the pores of the MCs, the AuNP-flecked MCs were pelleted from suspension, then dried in an oven at 80°C, sliced by ultra-microtomy, and characterized by SEM and Nano-SIMS (nanoscale secondary ion mass spectrometry). The process of oven drying was found to fuse the microcarrier interfaces, as can be observed in Fig. 4. Complete separation of microcarriers could not be achieved, even though better results were obtained than when drying in the oven. The nanoparticles could be seen in SEM to be restricted to the interface between microcarriers, with no evidence of an internal distribution within the MC (see Fig. 5). Nano-SIMS measurements of the elemental distribution of $^{197}$Au on microtome slice confirm this finding. Therefore, the NP-covered MC can be represented by a simple geometric model, with AuNPs evenly distributed on the exterior of the swollen MCs.

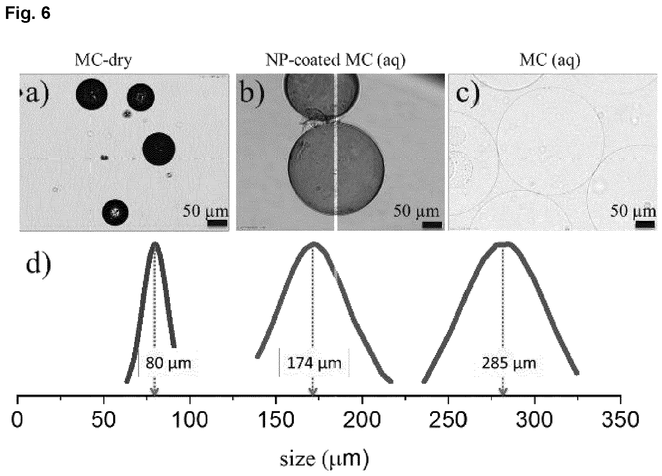

Fig. 6 shows the size distributions (d) of naked MCs in dry state (a), of naked MCs in water (c) and of AuNP-coated MCs. The swelling from dry to fully hydrated state (+260% in terms of diameter) in water is higher than the swelling that is achieved when putting dry MCs into a AuNP suspension (+115% in terms of diameter). The lower swelling in AuNP suspension can be the result of screening of electrostatic repulsion resulting from high ionic strength.

Taking RSA as a model for the electrostatic assembly process of the AuNPs on the MC surface, the concentration of NPs (in NPs per MC) necessary to reach the jamming limit can be calculated as:

$$[NP]_{ideal} = \frac{54.7\% \cdot S_{MC}}{S_{NP}} = 2.2 \left(\frac{R}{r}\right)^2, \qquad \text{(Eqn. 1)}$$

where $S_{MC} = 4\pi R^2$ is the surface area of the MCs with R being the MC radius and $S_{NP} = \pi r^2$ is the footprint area of the gold nanoparticles with r being the NP radius. With $r = 6$ $nm$ and $R = 86$ $\mu m$, $[NP]_{ideal} = 4.5 \cdot 10^8$.

To coat the MCs with gold nanoparticles, the nanoparticle concentrations in the suspension ($[NP]_{ution}$) was increased systematically in relation to the nanoparticle concentrations corresponding to a monolayer coverage ($[NP]_{ideal}$, Eqn 1). The concentration of nanoparticles in solution ($[NPs]_{ution}$) is defined as the number of nanoparticles in solution available per microcarrier and is calculated by dividing the total number of nanoparticles by the total number of microcarriers present in the same volume. The total number of nanoparticles in the suspension can be estimated from the ratio of the mass of gold in solution to the mass of one gold nanoparticle. The mass of gold in solution is known from the molar concentrations of gold salt used during synthesis, all of which is assumed to be reduced to gold. The mass per gold particle is obtained by approximating the NPs as spheres, with the diameter corresponding to the peak of the size distribution (cf. Fig. 3), and assuming the density of gold NPs to be the same as bulk gold. The number of MCs per unit volume was derived from the manufacturer's specification of the number of MCs per gram of dry weight, which was $6.8 \cdot 10^6$ $g^{-1}$ for Cytodex™-1, and the mass of dry powder employed.

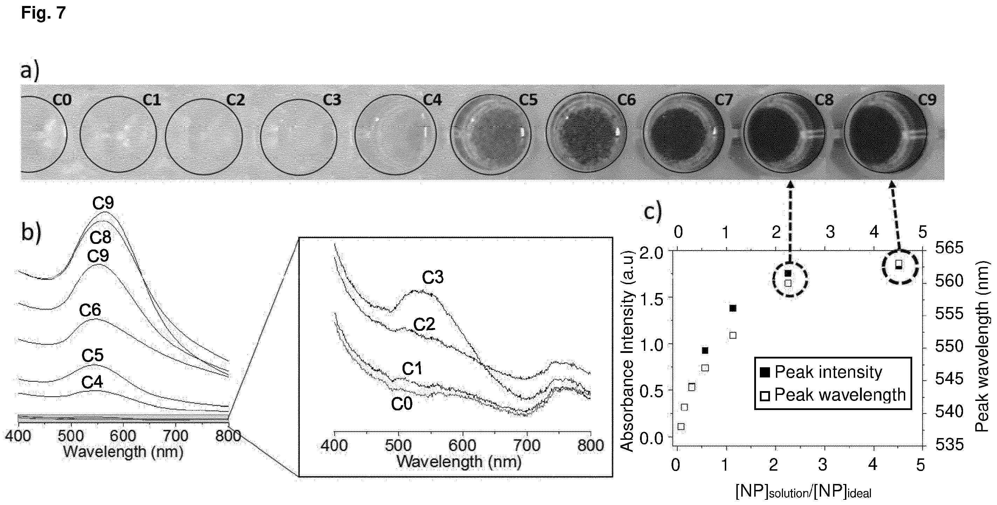

Cytodex™-1 MCs coated with AuNPs are easily distinguished from Cytodex-1 without any coating, since the naked MCs are transparent in water, the coated MCs appear purple with intensity depending on nanoparticle coverage (Fig. 7 a)). The absorbance spectra of the microcarriers coated with Au nanoparticles a systematic increase in the peak intensity of the localized surface plasmon resonance band of gold particles, as a function of nanoparticle coverage (Fig. 7 b) and c)). The increase in peak intensity is also associated with a red shift of the peak wavelength (Fig. 7 b) and c)). Both changes plateau at the saturated coverage of gold particles (Fig. 7 c)). The red shift in peak position is in line with expectations for plasmonic coupling between adjacent particles, the opportunity for which increases with particle coverage. Saturated coverage was achieved when the nanoparticle concentration amounted to three or more times $[NP]_{ideal}$. UV-visible spectra of the microcarriers were obtained using a TECAN Infinite M1000 PRO plate reader and were recorded in the 250 to 1000 nm range. Greiner CELLSTAR™ 96-well plate was used as plate reader. The sample was prepared as follows: 200 $\mu$l of each MCs suspension (C0-C9) were added into the microplate wells. The table below indicates the reagents used for functionalization of microcarriers with AuNPs, namely the mass of Cytodex-1, the volume of AuNPs suspension (produced as indicated above) and the volume of water added to reach a total volume of 2 ml each time.

| Sample name | Mass of MCs [mg] | Volume of AuNP solution [ml] | Volume of water [ml] |
|---|---|---|---|
| $C_0$ | 10 | 0.000 | 2.000 |
| $C_1$ | 10 | 0.060 | 1.940 |
| $C_2$ | 10 | 0.120 | 1.880 |
| $C_3$ | 10 | 0.240 | 1.760 |
| $C_4$ | 10 | 0.480 | 1.520 |
| $C_5$ | 10 | 0.960 | 1.040 |
| $C_6$ | 10 | 1.920 | 0.080 |
| $C_7$ | 5 | 1.920 | 0.080 |
| $C_8$ | 2.5 | 1.920 | 80 |
| $C_9$ | 1 | 1.536 | 464 |

The different masses of commercial MC powder were weighted directly in nine different Eppendorf tubes. The respective volumes of AuNP suspension and water were then added. The MCs were incubated for 3 hours. After incubation, the MCs had sedimented and the supernatant was removed. The MCs were then washed three times by:

   ◦ adding water in the Eppendorf tube and pipetting up and down to wash all the MCs in the solution;

   ◦ centrifuging at 1300 g during 2 minutes; and

   ◦ removing the supernatant.

The AuNP-coated MCs were then characterized as to their spectra.

Inelastic scattering properties were evaluated by Raman spectroscopy. Raman Scattering spectra were recorded in the 100 to 4000 $cm^{-1}$ extended range using Renishaw inVia™ Raman microscope equipped with a high-power near-IR laser diode working at 785 nm and a visible laser diode working at 633 nm. Before acquisition of the spectra, an optical microscope (Olympus; objective, x50L) was used to focus the laser beam. The laser output power was 0.9 mW (10%). For each spectrum, 6 accumulations of 5 seconds were recorded. To ensure a representative characterization of surfaces, multiple measurements were taken on both different parts of the same microcarrier and different microcarriers. Results were analyzed using WiRE™ and OriginLab™ software. Spectra acquired were smoothed and normalized. The sample was prepared as follows: some MCs were placed on a carbon tab.

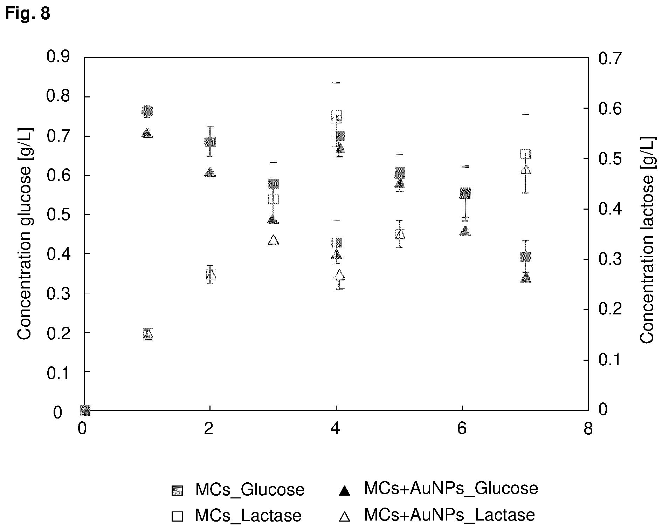

Cell culture tests were carried out with AuNP-coated MCs (Cytodex™-1) and the uncoated version thereof. As shown in Fig. 8, cell kinetic growth was monitored by measuring the time evolution of the concentration in glucose and lactate on a daily basis. Glucose is the main source of energy for the cell and lactate is one of the metabolite by-products of the glucose metabolism. Glucose and lactate concentrations are thus important indicators of cell growth and metabolic activity. About 50-100% of glucose is usually converted to lactate in cell culture fermentation. Cells on AuNP-coated Cytodex™-1 MCs consumed glucose in the same way as those on the uncoated Cytodex™-1 MCs. The consumption rate of glucose slowed down when glucose became limited and the production rate of lactate decreased consequently. No significant difference between commercial Cytodex™-1 and AuNPs-coated Cytodex™-1 MCs was noted.

Before cell culture, Cytodex™-1 MCs should be swelled in PBS (phosphate-buffered saline) and autoclaved at 120°C to sterilize them. However, the Cytodex-1 MCs were not autoclaved before attachment of gold nanoparticles. Since after AuNP attachment potentially impacts the interaction of gold nanoparticles with microcarriers, cell culture was performed on both autoclaved and non-autoclaved AuNP-coated Cytodex™-1.

Quantification of cell viability was effected by fluorescent microscopy. The cells (human mesenchymal stem cells) were mixed with Calcein AM and Ethidium Homodimer-1 (EthD-1) molecules to stain live (green) and dead (red) cells, respectively. It was found that cells colonized AuNP-coated Cytodex™-1 in a larger extent when they were not autoclaved. Hence, autoclaving has an impact on the microcarriers that inhibits adhesion of cells. However, the interaction between AuNPs and Cytodex™-1 MCs did not seem to be affected: the microcarriers were still opaque after autoclaving, confirming the presence of AuNPs. Without wanting to be bound by theory, the inventors indicated that one reason for the lower cell adhesion might be the break of the electrostatic interaction between the gold nanoparticles and the citrate (stabilizer of the gold nanoparticles) due to high temperature. The removal of the negatively charged citrate molecules would change the overall charge on the surface of the microcarrier and thus affect the interaction with the cell.

It was furthermore tested whether cells cultured on commercial microcarriers would migrate to plasmonic-nanoparticle-flecked microcarriers. The migration assay comprised adding (non-autoclaved) AuNP-coated Cytodex™-1 into a cell culture on naked but otherwise identical Cytodex™-1 microcarriers.

2 ml of Cytodex™-1 suspension (20 g/l) and 25 ml of cell culture medium were added into a 125 ml Erlenmeyer flask. The MCs were kept in that container during 1 hour at 37°C and 5% $CO_2$. Cells were detached and harvested from their container flasks by trypsinization. They were added into the Erlenmeyer already containing the microcarriers ($0.8 \cdot 10^5$ cells/ml). Cells and MCs were incubated in the cell culture medium to allow adhesion of the cells on the MCs. After 1 hour, cells anchored on microcarriers were put in agitation at 70 RPM (37°C; 5% $CO_2$). Samples of cell culture medium were withdrawn on a daily basis to measure glucose and glutamine consumption, as well as lactate, ammonium and lactate dehydrogenase production. Cell viability was assessed by *fluorescence microscopy.* To do this, cells were mixed with DAPI, Calcein AM and Ethidium Homodimer-1 (EthD-1) molecules to stain cells, live cells and dead cells, respectively.

On day 4, 50% of the cell culture medium was changed. Microcarriers coated with gold nanoparticles) were added to the Cytodex™-1 culture flask on day 4. After 1 hour of incubation, agitation was restarted (70 RPM). Quantification of cells was made on a daily basis with fluorescent microscopy. The cells were mixed with DAPI to stain cells. Cells were able to move towards the new carriers, confirming the capability of the modified microcarriers to support cell-expansion functions.

To summarize, the optical properties of the plasmonic-nanoparticle-coated microcarriers correlate with the gold nanoparticle coverage and indicate strong plasmonic coupling at saturated coverage. The plasmonic-NP-coated MCs open the possibility to detect smaller organic molecules using SERS or other plasmonic sensing techniques. Comparison of the plasmonic-NP-coated MCs with their un-coated counterparts showed similar cell growth kinetics, and migration of human mesenchymal stem cells, giving first evidence of the retention of cell-expansion functionality. The plasmonic microcarriers reported here, pave the way forward for plasmonic sensing of changes at the interface between microcarriers and biological media, and for monitoring changes to the biological cells at the different phases of cell-expansion, and to potentially enable plasmonic release of cells by plasmonic heating effects.

While specific embodiments have been described herein in detail, those skilled in the art will appreciate that various modifications and alternatives to those details could be developed in light of the overall teachings of the disclosure. Accordingly, the particular arrangements disclosed are meant to be illustrative only and not limiting as to the scope of the invention, which is to be given the full breadth of the appended claims and any and all equivalents thereof.

## Claims

1. A cell culture microcarrier bead (10), comprising a bead body (12) having its surface flecked with plasmonic nanoparticles (14), the bead body having a diameter in the range from 50 $\mu$m to 1 mm, the bead body comprising a swollen or unswollen hydrogel.

2. The microcarrier bead as claimed in 1, wherein the plasmonic nanoparticles comprise plasmonic metal nanoparticles.

3. The microcarrier bead as claimed in claim 1, wherein the plasmonic metal nanoparticles comprise gold nanoparticles (114).

4. The microcarrier bead as claimed in any one of claims 1 to 3, wherein the plasmonic nanoparticles are stabilized with a capping agent, e.g. citrate.

5. The microcarrier bead as claimed in any one of claims 1 to 4, wherein the plasmonic nanoparticles are spherical.

6. The microcarrier bead as claimed in any one of claims 1 to 5, wherein the plasmonic nanoparticles have a diameter in the range from 2 to 200 nm.

7. The microcarrier bead as claimed in any one of claims 1 to 6, wherein the ratio of the diameter of the bead body to the diameter of the plasmonic nanoparticles lies in the range from 250 to 25000.

8. A cell culture reactor, containing a cell culture medium and microcarrier beads as claimed in any one of claims 1 to 7.

9. A method for observing living cells on a microcarrier bead as claimed in any one of claims 1 to 7, comprising:

   illuminating the microcarrier bead with probe light so as to excite localized surface plasmons in the plasmonic nanoparticles,
   and detecting light emitted from the plasmonic nanoparticles in response to the illumination.

10. The method for observing living cells as claimed in claim 9, wherein the detection is based on one or more of the following techniques: localized surface plasmon resonance (LSPR), Surface-enhanced Raman spectroscopy (SERS), fluorescence and second-harmonic generation.

11. A method for packing nanoparticles (114) on a cell culture microcarrier (112) bead, the method comprising:

attaching nanoparticles on a surface of said cell culture microcarrier bead in a swollen state,
reducing the volume of the cell culture microcarrier bead and thereby causing the nanoparticles to move closer together, wherein the cell culture microcarrier bead comprises a hydrogel and wherein said volume reduction is effected by at least partial dehydration of the hydrogel.

12. The method as claimed in claim 11, wherein the nanoparticles are plasmonic nanoparticles, e.g. plasmonic metal nanoparticles.

13. A method for culturing living cells (16) on a microcarrier bead (10) as claimed in any one of claims 1 to 7, wherein release of cells from the microcarrier bead is stimulated by exciting localized surface plasmon resonance in said plasmonic nanoparticles.

14. The method as claimed in claim 13, wherein said release of cells is stimulated by plasmonic heating of said nanoparticles.

**Patentansprüche**

1. Zellkulturmikroträgerperle (10), umfassend einen Perlenkörper (12), dessen Oberfläche mit plasmonischen Nanopartikeln (14) besät ist, wobei der Perlenkörper einen Durchmesser im Bereich von 50 $\mu$m bis 1 mm aufweist und ein gequollenes oder nicht gequollenes Hydrogel umfasst.

2. Mikroträgerperle nach Anspruch 1, wobei die plasmonischen Nanopartikel plasmonische Metallnanopartikel umfassen.

3. Mikroträgerperle nach Anspruch 1, wobei die plasmonischen Metallnanopartikel Goldnanopartikel (114) umfassen.

4. Mikroträgerperle nach einem der Ansprüche 1 bis 3, wobei die plasmonischen Nanopartikel mit einem Verkappungsmittel, z. B. Citrat, stabilisiert sind.

5. Mikroträgerperle nach einem der Ansprüche 1 bis 4, wobei die plasmonischen Nanopartikel kugelförmig sind.

6. Mikroträgerperle nach einem der Ansprüche 1 bis 5, wobei die plasmonischen Nanopartikel einen Durchmesser im Bereich von 2 bis 200 nm aufweisen.

7. Mikroträgerperle nach einem der Ansprüche 1 bis 6, wobei das Verhältnis des Durchmessers des Perlenkörpers zum Durchmesser der plasmonischen Nanopartikel im Bereich von 250 bis 25000 liegt.

8. Zellkulturreaktor, enthaltend ein Zellkulturmedium und Mikroträgerperlen nach einem der Ansprüche 1 bis 7.

9. Verfahren zur Beobachtung lebender Zellen auf einer Mikroträgerperle nach einem der Ansprüche 1 bis 7, umfassend:

Beleuchten des Mikroträgerperles mit Prüflicht, um lokalisierte Oberflächenplasmonen in den plasmonischen Nanopartikeln anzuregen,
und Detektieren von Licht, das von den plasmonischen Nanopartikeln als Reaktion auf die Beleuchtung emittiert wird.

10. Verfahren zur Beobachtung lebender Zellen gemäß Anspruch 9, wobei die Detektion auf einer oder mehreren der folgenden Techniken basiert: lokalisierte Oberflächenplasmonenresonanz (LSPR), oberflächenverstärkte Ramanspektroskopie (SERS), Fluoreszenz und Erzeugung der zweiten Harmonischen.

**11.** Verfahren zum Laden von Nanopartikeln (114) auf eine Zellkulturmikroträgerperle (112), wobei das Verfahren umfasst:

das Anbringen von Nanopartikeln an einer Oberfläche der Zellkulturmikroträgerperle in einem gequollenen Zustand,
das Verringern des Volumens der Zellkulturmikroträgerperle und dadurch das Bewirken, dass sich die Nanopartikel einander annähern, wobei die Zellkulturmikroträgerperle ein Hydrogel umfasst und wobei die Volumenverringerung durch zumindest teilweise Dehydratisierung des Hydrogels bewirkt wird.

**12.** Verfahren nach Anspruch 11, wobei die Nanopartikel plasmonische Nanopartikel sind, z. B. plasmonische Metallnanopartikel.

**13.** Verfahren zur Kultivierung lebender Zellen (16) auf einer Mikroträgerperle (10) nach einem der Ansprüche 1 bis 7, wobei die Ablösung von Zellen von der Mikroträgerperle durch Anregung einer lokalisierten Oberflächenplasmonenresonanz in den plasmonischen Nanopartikeln stimuliert wird.

**14.** Verfahren nach Anspruch 13, wobei die Ablösung von Zellen durch plasmonische Erwärmung der Nanopartikel stimuliert wird.

## Revendications

**1.** Bille micro-support pour culture cellulaire (10), comprenant un corps de bille (12) dont la surface est parsemée de nanoparticules plasmoniques (14), le corps de bille ayant un diamètre compris dans la plage de 50 $\mu$m à 1 mm, le corps de bille comprenant un hydrogel gonflé ou non gonflé.

**2.** Bille micro-support selon la revendication 1, dans laquelle les nanoparticules plasmoniques comprennent des nanoparticules métalliques plasmoniques.

**3.** Bille micro-support selon la revendication 1, dans laquelle les nanoparticules en métalliques plasmoniques comprennent des nanoparticules d'or (114).

**4.** Bille micro-support selon l'une quelconque des revendications 1 à 3, dans laquelle les nanoparticules plasmoniques sont stabilisées à l'aide d'un agent de coiffage, par exemple du citrate.

**5.** Bille micro-support selon l'une quelconque des revendications 1 à 4, dans laquelle les nanoparticules plasmoniques sont sphériques.

**6.** Bille micro-support selon l'une quelconque des revendications 1 à 5, dans laquelle les nanoparticules plasmoniques ont un diamètre compris dans la plage de 2 à 200 nm.

**7.** Bille micro-support selon l'une quelconque des revendications 1 à 6, dans laquelle le rapport entre le diamètre du corps de la bille et le diamètre des nanoparticules plasmoniques se situe dans la plage de 250 à 25000.

**8.** Réacteur de culture cellulaire, contenant un milieu de culture cellulaire et des billes micro-supports selon l'une quelconque des revendications 1 à 7.

**9.** Procédé d'observation de cellules vivantes sur une bille micro-support selon l'une quelconque des revendications 1 à 7, comprenant :

l'illumination de la bille micro-support avec une lumière de sondage de manière à exciter des plasmons de surface localisés dans les nanoparticules plasmoniques,
et la détection de la lumière émise par les nanoparticules plasmoniques en réponse à l'illumination.

**10.** Procédé d'observation de cellules vivantes selon la revendication 9, dans lequel la détection repose sur une ou plusieurs des techniques suivantes : la résonance de plasmon de surface localisé (LSPR), la spectroscopie Raman améliorée en surface (SERS), la fluorescence et la génération de seconde harmonique.

11. Procédé pour charger des nanoparticules (114) sur une bille micro-support pour culture cellulaire (112), le procédé comprenant :

la fixation de nanoparticules sur une surface de ladite bille micro-support pour culture cellulaire à l'état gonflé, la réduction du volume de la bille micro-support pour culture cellulaire, provoquant ainsi le rapprochement des nanoparticules, dans lequel la bille micro-support pour culture cellulaire comprend un hydrogel et dans lequel ladite réduction de volume est effectuée par une déshydratation au moins partielle de l'hydrogel.

12. Procédé selon la revendication 11, dans lequel les nanoparticules sont des nanoparticules plasmoniques, par exemple des nanoparticules métalliques plasmoniques.

13. Procédé de culture de cellules vivantes (16) sur une bille micro-support (10) selon l'une quelconque des revendications 1 à 7, dans lequel la libération des cellules de la bille micro-support est stimulée par l'excitation d'une résonance de plasmon de surface localisé dans lesdites nanoparticules plasmoniques.

14. Procédé selon la revendication 13, dans lequel ladite libération des cellules est stimulée par un échauffement plasmonique desdites nanoparticules.

**Fig. 1**

**Fig. 2**

**Fig. 3**

**Fig. 4**

**Fig. 5**

**Fig. 6**

Fig. 7

**Fig. 8**